# EUROPÄISCHE PATENTSCHRIFT

(11) **EP 1 664 102 B1**
(45) Veröffentlichungstag und Bekanntmachung des Hinweises auf die Patenterteilung: **16.11.2011**
(21) Anmeldenummer: 04764797.9
(22) Anmeldetag: 03.09.2004
(51) Int. Cl.: C07K 14/435, C12N 5/10, C12N 15/12, C12Q 1/68, G01N 33/50, G01N 33/533

(54) **ISOLIERTES PHOTOPROTEIN MTCLYTIN, SOWIE DESSEN VERWENDUNG**
ISOLATED PHOTOPROTEIN MTCLYTIN, AND USE THEREOF
PHOTOPROTEINE MTCLYTINE ISOLEE ET SON UTILISATION

(30) Priorität: 16.09.2003 DE 10342670
(43) Veröffentlichungstag der Anmeldung: 07.06.2006
(73) Patentinhaber: AXXAM S.p.A., 20132 Milano (IT)
(72) Erfinder: GOLZ, Stefan, 45326 Essen (DE); MARKOVA, Svetlana, Krasnoyarsk, 660036 (RU); BURAKOVA, Ludmila, Sosnovoborsk, 662500 (RU); FRANK, Ludmila, Krasnoyarsk, 660036 (RU); VYSOTSKI, Eugene, Krasnoyarsk, 660100 (RU)
(74) Vertreter: Banfi, Paolo
(86) Internationale Anmeldenummer: PCT/EP2004/009843
(87) Internationale Veröffentlichungsnummer: WO 2005/035559

(56) Entgegenhaltungen:
- WO-A-91/01305
- WO-A-03/006497
- DATABASE UniProt 1. Oktober 1994 (1994-10-01), INOUYE S. ET AL.: "Clytin precursor (Phialidin)" XP002300448 Database accession no. Q08121 -& INOUYE S ET AL: "CLONING AND SEQUENCE ANALYSIS OF CDNA FOR THE CA2+-ACTIVATED PHOTOPROTEIN, CLYTIN" FEBS LETTERS, ELSEVIER SCIENCE PUBLISHERS, AMSTERDAM, NL, Bd. 315, Nr. 3, Januar 1993 (1993-01), Seiten 343-346, XP001180448 ISSN: 0014-5793 in der Anmeldung erwähnt
- DATABASE EMBL 10. März 2002 (2002-03-10), MARKOVA S. V. ET AL.: "Obelia geniculata apoobelin mRNA, complete cds" XP002305433 Database accession no. AF394688 -& MARKOVA SVETLANA V ET AL: "Obelin from the bioluminescent marine hydroid Obelia geniculata: Cloning, expression, and comparison of some properties with those of other Ca2+-regulated photoproteins" BIOCHEMISTRY, AMERICAN CHEMICAL SOCIETY. EASTON, PA, US, Bd. 41, Nr. 7, 19. Februar 2002 (2002-02-19), Seiten 2227-2236, XP002232863 ISSN: 0006-2960
- DUNSTAN S L ET AL: "Cloning and expression of the bioluminescent photoprotein pholasin from the bivalve mollusc Pholas dactylus." THE JOURNAL OF BIOLOGICAL CHEMISTRY. 31 MAR 2000, Bd. 275, Nr. 13, 31. März 2000 (2000-03-31), Seiten 9403-9409, XP002305955 ISSN: 0021-9258

### SEQUENCE LISTING

<110> Bayer AG, BHC
<120> Isoliertes Photoprotein mtClytin, sowie dessen Verwendung
<130> Le A 36 839
<160> 6
<170> PatentIn version 3.1
<210> 1
   <211> 912
   <212> DNA
   <213> Clytia gregaria
<400> 1
   gacagataaa aaattcactc cttagattat ttagtgaata agagaaaaaa aggataagaa     60
   atcaagatgc aaaggtttac aaatcgtctt ctttccatgt cggctttacg tgcaagatca    120
   agattgcaac gcacggcaaa ttttcacacc agcatactct tggctacaga ttcaaaatac    180
   gcggtcaaac tcgatcctga ttttgcaaat ccaaaatgga tcaacagaca caaatttatg    240
   ttcaactttt tggacataaa cggtaagggg aaaatcacat tagatgaaat cgtctccaaa    300
   gcttcagacg acatttgtgc taaactggat gcaacaccag aacagaccaa acgtcaccag    360
   gatgctgttg aagccttttt caagaaaatg ggcatggatt atggtaaaga agttgcattc    420
   ccagaattta ttaagggatg ggaagagttg gccgaacacg acttggaact ctggtctcaa    480
   aacaaaagta cattgatccg tgaatgggga gatgctgttt tcgacatttt cgacaaagac    540
   gcaagtggct caatcagttt agacgaatgg aaggcttacg gacgaatctc tggaatctgt    600
   ccatcagacg aagacgctga gaagacgttc aaacattgtg atttggacaa cagtggcaaa    660
   cttgatgttg atgagatgac caggcaacat ttaggcttct ggtacacatt ggatccaact    720
   tctgatggtc tttatggcaa ttttgttccc taagaagcgt tcagttaaaa acgctaaaca    780
   ttgttcagtt gtaaaattat attcattttc atttcgtaaa attagtattt ataaatttgt     840
   atcataaatt gtatccatgt tgtagactaa ataagactcg gcaaaaaaaa aaaaaaaaaa    900
   aaaaaaaaaa aa                                                        912
<210> 2
   <211> 228
   <212> PRT
   <213> Clytia gregaria
<400> 2
   Met Gln Arg Phe Thr Asn Arg Leu Leu Ser Met Ser Ala Leu Arg Ala
   1               5                   10                  15
   Arg Ser Arg Leu Gln Arg Thr Ala Asn Phe His Thr Ser Ile Leu Leu
               20                  25                  30
   Ala Thr Asp Ser Lys Tyr Ala Val Lys Leu Asp Pro Asp Phe Ala Asn
           35                  40                  45
   Pro Lys Trp Ile Asn Arg His Lys Phe Met Phe Asn Phe Leu Asp Ile
       50                  55                  60
   Asn Gly Lys Gly Lys Ile Thr Leu Asp Glu Ile Val Ser Lys Ala Ser
   65                  70                  75                  80
   Asp Asp Ile Cys Ala Lys Leu Asp Ala Thr Pro Glu Gln Thr Lys Arg
                   85                  90                  95
   His Gln Asp Ala Val Glu Ala Phe Phe Lys Lys Met Gly Met Asp Tyr
               100                 105                 110
   Gly Lys Glu Val Ala Phe Pro Glu Phe Ile Lys Gly Trp Glu Glu Leu
           115                 120                 125
   Ala Glu His Asp Leu Glu Leu Trp Ser Gln Asn Lys Ser Thr Leu Ile
       130                 135                 140
   Arg Glu Trp Gly Asp Ala Val Phe Asp Ile Phe Asp Lys Asp Ala Ser
   145                 150                 155                 160
   Gly Ser Ile Ser Leu Asp Glu Trp Lys Ala Tyr Gly Arg Ile Ser Gly
                   165                 170                 175
   Ile Cys Pro Ser Asp Glu Asp Ala Glu Lys Thr Phe Lys His Cys Asp
               180                 185                 190
   Leu Asp Asn Ser Gly Lys Leu Asp Val Asp Glu Met Thr Arg Gln His
           195                 200                 205
   Leu Gly Phe Trp Tyr Thr Leu Asp Pro Thr Ser Asp Gly Leu Tyr Gly
       210                 215                 220
   Asn Phe Val Pro
   225
<210> 3
   <211> 16
   <212> PRT
   <213> Clytia gregaria
<400> 3
   Met Gln Arg Phe Thr Asn Arg Leu Leu Ser Met Ser Ala Leu Arg Ala
   1               5                   10                  15
<210> 4
   <211> 48
   <212> DNA
   <213> Clytia gregaria
<400> 4
   atgcaaaggt ttacaaatcg tcttctttcc atgtcggctt tacgtgca 48
<210> 5
   <211> 791
   <212> DNA
   <213> Clytia gregaria
<400> 5
   gatctcagct caacttgcaa taagtatcag atcaaatttt gcaactcaaa gcaaatcatc 60
   aacttcatca taatgactga cactgcttca aaatacgctg tcaaactcaa gaccaacttt 120
   gaagatccaa aatgggtcaa cagacacaaa tttatgttca actttttgga cattaacggc 180
   aacggaaaaa tcactttgga tgaaattgtc tccaaagctt cggatgacat ttgcgccaaa 240
   cttggagcta caccagctca aacccaacgt catcaggaag ctgttgaagc tttcttcaag 300
   aagattggtt tggattatgg caaagaagtc gaattcccag ctttcgttaa cggatggaaa 360
   gaactggcca aacatgactt gaaactttgg tcccaaaaca agaaatcttt gatccgcaat 420
   tggggagaag ctgtattcga cattttcgac aaggacggaa gtggctcaat cagtttggac 480
   gaatggaaaa catacggagg aatctctgga atctgtccat cagacgaaga cgctgaaaag 540
   accttcaaac attgcgattt ggacaacagt ggcaaacttg atgttgacga gatgaccaga 600
   caacatttgg gattctggta caccttggac cctaacgctg atggtcttta tggcaacttt 660
   gtcccttaaa aacttttttt gctgtaaatt ctttacgggt tattttttca taattgtcat 720
   ttgattttaa ctttgtttcg gaaaatgaaa aatattcttt attcagaaaa aaaaaaaaaa 780
   aaaaaaaaaa a 791
<210> 6
   <211> 198
   <212> PRT
   <213> Clytia gregaria
<400> 6
   Met Thr Asp Thr Ala Ser Lys Tyr Ala Val Lys Leu Lys Thr Asn Phe
   1 5 10 15
   Glu Asp Pro Lys Trp Val Asn Arg His Lys Phe Met Phe Asn Phe Leu
   20 25 30
   Asp Ile Asn Gly Asn Gly Lys Ile Thr Leu Asp Glu Ile Val Ser Lys
   35 40 45
   Ala Ser Asp Asp Ile Cys Ala Lys Leu Gly Ala Thr Pro Ala Gln Thr
   50 55 60
   Gln Arg His Gln Glu Ala Val Glu Ala Phe Phe Lys Lys Ile Gly Leu
   65 70 75 80
   Asp Tyr Gly Lys Glu Val Glu Phe Pro Ala Phe Val Asn Gly Trp Lys
   85 90 95
   Glu Leu Ala Lys His Asp Leu Lys Leu Trp Ser Gln Asn Lys Lys Ser
   100 105 110
   Leu Ile Arg Asn Trp Gly Glu Ala Val Phe Asp Ile Phe Asp Lys Asp
   115 120 125
   Gly Ser Gly Ser Ile Ser Leu Asp Glu Trp Lys Thr Tyr Gly Gly Ile
   130 135 140
   Ser Gly Ile Cys Pro Ser Asp Glu Asp Ala Glu Lys Thr Phe Lys His
   145 150 155 160
   Cys Asp Leu Asp Asn Ser Gly Lys Leu Asp Val Asp Glu Met Thr Arg
   165 170 175
   Gln His Leu Gly Phe Trp Tyr Thr Leu Asp Pro Asn Ala Asp Gly Leu
   180 185 190
   Tyr Gly Asn Phe Val Pro
   195

## Beschreibung

Die Erfindung betrifft das Photoprotein mtClytin, dessen Nukleotid- und Aminosäuresequenz, sowie die Aktivität und Verwendung des Photoproteins mtClytin.

### Photoproteine

Als Biolumineszenz bezeichnet man das Phänomen der Lichterzeugung durch Lebewesen. Sie ist das Ergebnis von biochemischen Reaktionen in Zellen, bei denen die chemische Energie in Form von Lichtquanten abgegeben wird (sog. kalte Emission durch Chemolumineszenz). Derartig erzeugtes Licht ist monochromatisch, denn es wird bei einem diskreten Elektronen-Übergang abgestrahlt, kann aber durch sekundäre Leuchtfarbstoffe (z.B. fluoreszierende Proteine bei Leuchtquallen der Gattung Aequora) in längerwellige Spektralbereiche verschoben werden.

Die biologische Funktion ist vielfältig: In der Meerestiefe zwischen 200 und 1000 m (Mesopelagial) leuchten rund 90 % aller Lebewesen. Die Leuchtsignale werden hier zur Partnerwerbung, Täuschung und als Köder eingesetzt. Auch Glühwürmchen und Leuchtkäfer nutzen die Lichtsignale zur Partnersuche. Die Bedeutung des Leuchtens von Bakterien, Pilzen und einzelligen Algen ist dagegen unklar. Es wird vermutet, dass es zur Koordination von vielen Einzel-Individuen einer großen Population eingesetzt wird oder eine Art biologische Uhr darstellt.

Eine Vielzahl an Coelenteraten ist biolumineszent (Morin et al., 1974). Diese Organismen emittieren blaues oder grünes Licht. Das 1962 als erstes Licht produzierendes Protein identifizierte Aequorin aus Aequoria victoria (Shimomura et al., 1969) emittierte als isoliertes Protein ein blaues Licht und nicht grünes Licht wie phänotypisch beobachtet bei Aequoria victoria. Später konnte das grün fluoreszierende Protein (GFP) aus Aequoria victoria isoliert werden, das aufgrund der Anregung durch das Aequorin die Meduse phänotypisch grün erscheinen lässt (Johnson et al., 1962; Hastings et al., 1969; Inouye et al., 1994). Als weitere Photoproteine konnten noch Clytin (Inouye et al., 1993), Mitrocomin (Fagan et al., 1993) und Obelin (Illarionov et al., 1995) identifiziert und beschrieben werden.

**Tabelle 1: Übersicht über einige Photoproteine. Angegeben sind der Name, der Organismus aus dem das Protein isoliert worden ist und die Identifikationsnummer (Acc. No.) des Datenbankeintrages.**

| **Name** | **Organismus** | **Identifikations Nr.** |
|---|---|---|
| Obelin | Obelia geniculata | AAL86372 |
| Clytin | Clytia gregaria | CAA49754 |
| Aequorin | Aequorea macrodactyla | AAK02061 |
| Aequorin | Aequorea parva | AAK02060 |
| Mitrocomin | Mitrocoma cellularia | AAA29298 |
| Pholasin | Pholas dactylus | AAM18085 |
| ? | Symplectoteuthis oualaniensis | AX305029 |

**Tabelle 2: Übersicht über einige Photoproteine. Angegeben sind der Organismus aus dem das Protein isoliert worden ist, der Name des Photoproteins und eine Auswahl an Patenten bzw. Anmeldungen.**

| **Organismus** | **Fluoreszierendes Protein** | **Patent / Anmeldung** |
|---|---|---|
| Obelia geniculata | Obelin | WO03006497 |
| Clytia gregaria | Clytin | WO03006497 |
| Aequoria victoria | Aequorin | WO200168824 |
| | | US-0908909 |
| | | US 6,152,358 |
| | | JP-0176125 |
| Pholas dactylus | Pholasin | WO0028025 |
| | | GB-0024357 |

Biolumineszenz wird heute in der Technik vielfältig genutzt, z.B. in Form von Bio-Indikatoren für Umweltverschmutzung oder in der Biochemie zum empfindlichen Nachweis von Proteinen, zur Quantifizierung bestimmter Verbindungen oder als sogenannte "Reporter" bei der Untersuchung zellulärer Gen-Regulation.

Die Photoproteine unterscheiden sich nicht nur aufgrund ihrer Nukleotid- und Aminosäuresequenz, sondern auch aufgrund ihrer biochemischen und physikalischen Eigenschaften.

Es konnte gezeigt werden, dass durch die Veränderung der Aminosäuresequenz von Photoproteinen die physikalischen und biochemischen Eigenschaften verändert werden können. Beispiele von rautagenisierten Photoproteinen sind in der Literatur beschrieben (US 6,495,355; US 5,541,309; US 5,093,240; Shimomura et al., 1986).

Die Lichterzeugung durch die oben genannten Photoproteine erfolgt durch die Oxidation von Coelenterazin (Haddock et al., 2001; Jones et al., 1999).

Markeva SV et al, Biochemistry, Bd. 41, Mr. 7, 19 Februar 2002, Seiten 2227-2236 offenbart die Klonierung, Sequenzanalyse und Expression des Proteins Obelin aus dem biolumineszenten Meereshydroid Obelia geniculata. WO 91/01305 beschreibt biolumineszente Proteine und deren Verwendung als Marker/Reporter, *inter alia* die Photoproteine Aequorin und Obelin (Seite 7, Zeilen 17-21). Das Dokument beschreibt auch die Möglichkeit, die obengenannten Proteine einem Signalpeptide zu binden zur Transportierung in Zellorganellen, wie zum Beispiel Mitochondrien oder das endoplasmatische Retikulum (Seite 8, Zeilen 9-26).
Dunstan S L et al, Journal of Biological Chemistry, Bd. 275, No.: 13, 31/3/2000, Seiten 9403-9409, offenbart das Pholas dactylus Photoprotein, Pholasin, das ein natürliches Signalpeptide mit 20-Aminosäuren für die Sekretion des Proteins enthält (Zusammenfassung, Seite 9407, rechte Spalte, Zeilen 14-16).

### Reportersysteme

Als Reporter- oder Indikatorgen bezeichnet man generell Gene, deren Genprodukte sich mit Hilfe einfacher biochemischer oder histochemischer Methoden leicht nachweisen lassen. Man unterscheidet mindestens 2 Typen von Reportergenen.
1. Resistenzgene. Als Resistenzgene werden Gene bezeichnet, deren Expression einer Zelle die Resistenz gegen Antibiotika oder andere Substanzen verleiht, deren Anwesenheit im Wachstumsmedium zum Zelltod führt, wenn das Resistenzgen fehlt.
2. Reportergene. Die Produkte von Reportergenen werden in der Gentechnologie als fusionierte oder unfusionierte Indikatoren verwendet. Zu den gebräuchlichsten Reportergenen gehören die beta-Galaktosidase (Alam et al., 1990), alkalische Phosphatase (Yang et al., 1997; Cullen et al., 1992), Luciferasen und andere Photoproteine (Shinomura, 1985; Phillips GN, 1997; Snowdowne et al., 1984).

Als Lumineszenz bezeichnet man die Abstrahlung von Photonen im sichtbaren Spektralbereich, wobei diese durch angeregte Emittermoleküle erfolgt. Im Unterschied zur Fluoreszenz wird hierbei die Energie nicht von Außen in Form von Strahlung kürzerer Wellenlänge zugeführt.

Man unterscheidet Chemolumineszenz und Biolumineszenz. Als Chemolumineszenz bezeichnet man eine chemische Reaktion, die zu einem angeregten Molekül führt, das selbst leuchtet, wenn die angeregten Elektronen in den Grundzustand zurückkehren. Wird diese Reaktion durch ein Enzym katalysiert, spricht man von Biolumineszenz. Die an der Reaktion, beteiligten Enzyme werden generell als Luziferasen bezeichnet.

### Einordung der Spezies Clytia gregaria

Cnidaria→Leptomedusae→Campanulariidae→ Clytia gregaria

Die Spezies Clytia grenaria gehört zu den Cnidaria, speziell zu den Medusen. Der biolumineszente bzw. fluoreszente Phänotyp wurde bereits 1998 beschrieben (Ward et al., 1998).

### Isolierung der cDNA

Zur Untersuchung der Biolumineszenz-Aktivität der Spezies Clytia gregaria wurden Exemplare im Weißen Meer (Biologische Station Kartesh, Russland) gefangen und in flüssigem Stickstoff gelagert. Zur Erstellung der cDNA-Bibliotheken von Clytia gregaria, wurde die poly(a)+ RNA mit Hilfe des "Straight A" Isolationsmethode von Novagen (USA) isoliert.

Zur Herstellung der cDNA wurde eine RT-PCR durchgeführt. Hierzu wurden 1 µg RNA mit Reverser Transkriptase (Superscribt Gold II) nach folgendem Schema inkubiert:

| | | | | |
|---|---|---|---|---|
| PCR | 1. | 30 | Sekunden | 95°C |
| | 2. | 6 | Minuten | 68°C |
| | 3. | 10 | Sekunden | 95°C |
| | 4. | 6 | Minuten | 68°C |
| | | 17 | Zyklen von Schritt 4 nach Schritt 3 | |

Die Reaktionsprodukte wurden zur Inaktivierung der Polymerase für 30 Minuten bei 37°C mit Proteinase K inkubiert und die cDNA mit Ethanol präzipitiert. Die Expression-cDNA Bank wurde mit Hilfe des "SMART cDNA Library Construction Kits" der Firma Clontech (USA) nach Herstellerangaben durchgeführt. Die Klonierung erfolgte in den Expressionsvektor pTriplEx2 (Clontech; USA). Die Expressionsvektoren wurden durch Elektroporation in Bakterien des Stammes E. coli XL1-Blue transformiert.

Die Bakterien wurden auf LB-Nährböden plattiert und für 24 Stunden bei 37°C inkubiert. Anschließend wurde eine Replikaplattierung durchgeführt, indem die Bakterien mit Hilfe eines Nitrocellulosefilters auf eine weitere Nährbodenplatte übertragen wurden. Die Replikaplatte wurde wiederum für 24 Stunden bei 37°C inkubiert und die gewachsenen Bakterienkolonien in LB-Flüssigmedium übertragen. Nach der Zugabe von IPTG (Endkonzentration 0,1 mM) wurden die Bakterien für 4 Stunden bei 37°C auf einem Schüttler inkubiert. Die Bakterien wurden durch Zentrifugation geerntet und die Bakterienmasse in 0,5 ml Aufschlusspuffer (5 mM EDTA, 20 mM Tris-HCL pH 9,0) bei 0°C resuspendiert. Anschließend erfolgte der Aufschluss der Bakterien durch Ultraschall.

Die Lysate wurden nach der Zugabe von Coelenterazine (Endkonzentration 10E-07 M) bei 4°C für 3 Stunden inkubiert. Anschließend erfolgte die Messung der Biolumineszenz nach der Zugabe von Calziumchlorid (Endkonzentration 20 mM) im Luminometer.

Es wurde ein Photoprotein identifiziert. Das Photoprotein wurde als mtClytin bezeichnet. Im Folgenden wird das Photoprotein mtClytin im einzelnen dargestellt.

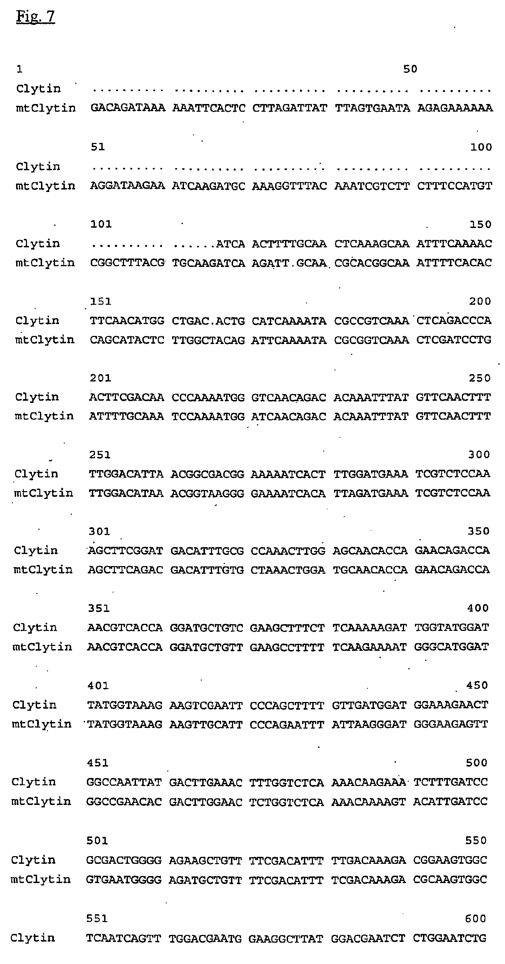

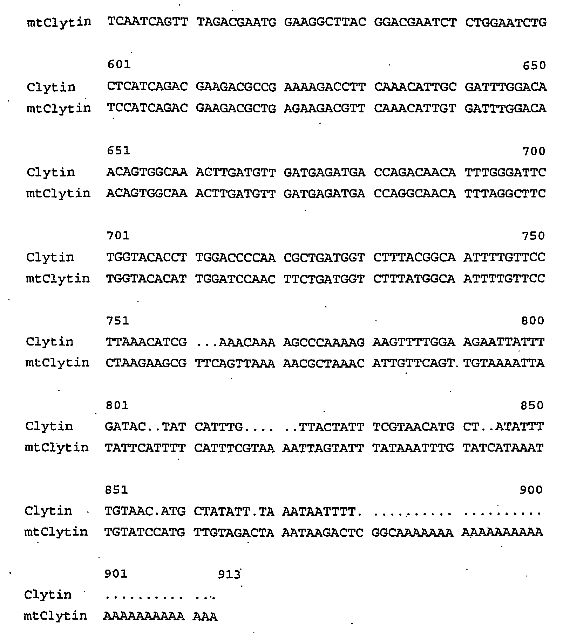

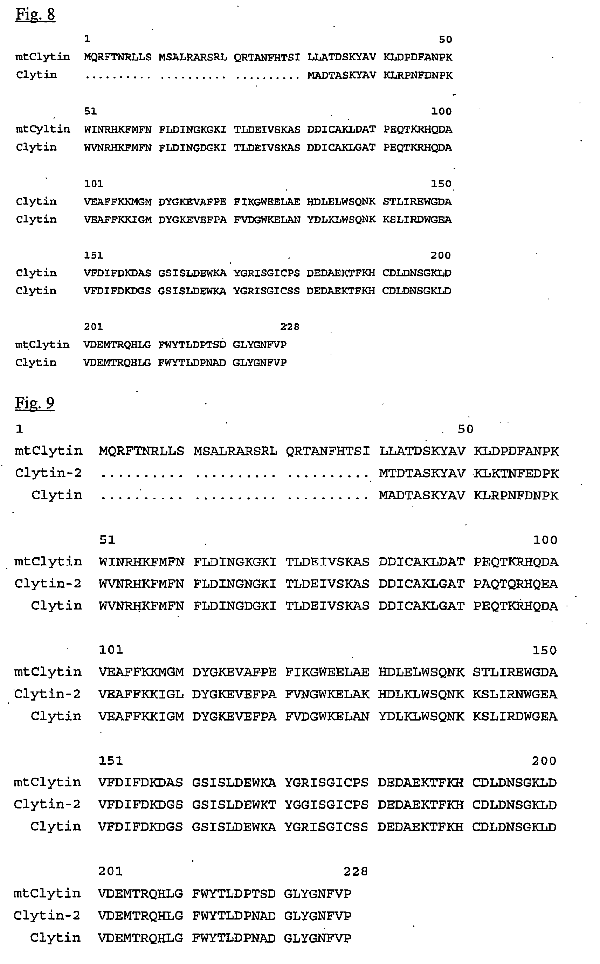

Das Photoprotein mtClytin zeigt die höchste Homologie auf Aminosäureebene zu Clytin aus Clytia gregaria mit einer Identität von 87 % und zu Obelin aus Obelia geniculata eine Identität von 77 % (gezeigt in Beispiel 8; Figur 8). Die Homologie von 87 % - in Bezug auf Clytin - ergibt sich am C-terminalen Ende des Proteins, wobei verteilt über das gesamte Protein mehrfache Aminosäureaustausche zu identifizieren sind. Auf Nukleinsäureebene liegt die Identität unter 30 % (gezeigt in Beispiel 7; Figur 7). Zum Sequenzvergleich wurde das BLAST-Verfahren verwendet (Altschul et al., 1997).

Das Photoprotein mtClytin besitzt ein Signalpeptid, das zur Translokation des Photoproteins in Mitochondrien führen kann. Die Identifizierung des Signalpeptides erfolgte durch das Computerprogramm MITOPROT (Claros et al., 1996) (gezeigt in Beispiel 10). Das durch MITOPROT ermittelte Signalpeptid ist in SEQ ID NO: 3 angegeben. Das Photoproctein mtClytin ist das erste Photoprotein, bei dem ein natürliches Signalpeptid zur Translokation in Mitochondrien identifiziert werden konnte.

Beschrieben sind auch funktionelle Äquivalente von mtClytin. Funktionelle Äquivalente sind solche Proteine, die vergleichbare physikochemische Eigenschaften haben und mindestens 70 % homolog sind zu SEQ ID NO: 2. Bevorzugt ist eine Homologie von mindestens 80 % oder 90 %. Besonders bevorzugt ist eine Homologie von mindestens 95 %.

Das Photoprotein mtClytin eignet sich als Reportergen für zelluläre Systeme speziell für Rezeptoren, für Ionenkanäle, für Transporter, für Transkriptionsfaktoren oder für induzierbare Systeme.

Das Signalpeptid von mtClytin eignet sich auch als Fusion mit Reportergenen als fusionierte Reportergen für zelluläre Systeme speziell für Rezeporen, für Ionenkanäle, für Transporter, für Transkriptionsfaktoren oder für induzierbare Systeme.

Das Photoprotein mtClytin eignet sich auch als Reportergen durch Markierung, Identifizierung und Charakterisierung von Zellorganellen speziell für Mitochondrien.

Das Photoprotein von mtClytin eignet sich auch als Reportergen zur Bestimung von Parametern innerhalb und außerhalb von Zellorganellen, speziell von Mitochondrien, speziell von Kalziumkonzentrationen.

Das Photoprotein mtClytin eignet sich als Reportergen in bakteriellen und eukaryotischen Systemen speziell in Säugerzellen, in Bakterien, in Hefen, in Bakulo, in Pflanzen.

Das Photoprotein mtClytin eignet sich als Reportergen für zelluläre Systeme in Kombination mit biolumineszenten oder chemolumineszenten Systemen, speziell Systemen mit Luziferasen, mit Oxygenasen, mit Phosphatasen.

Das Photoprotein mtClytin eignet sich als Fusionsprotein speziell für Rezeptoren, für Ionenkanäle, für Transporter, für Transkriptionsfaktoren, für Proteinasen, für Kinasen, für Phosphodiesterasen, für Hydrolasen, für Peptidasen, für Transferasen, für Membranproteine und für Glykoproteine.

Das Photoprotein mtClytin eignet sich zur Immobilisierung speziell durch Antikörper, durch Biotin, durch magnetische oder magnetisierbare Träger.

Das Photoprotein mtClytin eignet sich als Protein für Systeme des Energietransfers speziell der FRET- (Fluorescence Resonance Energy Transfer), BRET- (Bioluminescence Resonance Energy Transfer), FET (field effect transistors), FP (fluorescence polarisation), HTRF (Homogeneous time-resolved fluorescence) Systemen.

Das Photoprotein mtClytin eignet sich als Markierung von Substraten oder Liganden speziell für Proteasen, für Kinasen, für Transferasen.

Das Photoprotein mtClytin eignet sich zur Expression in bakteriellen Sytemen speziell zur Titerbestimmung, als Substrat für biochemische Systeme speziell für Proteinasen und Kinasen.

Das photoprotein mtClytin eignet sich als Marker speziell gekoppelt an Antikörper, gekoppelt an Enzyme, gekoppelt an Rezeptoren, gekoppelt an Ionenkanäle und andere Proteine.

Das Photoprotein mtClytin eignet sich als Reportergen bei der pharmakologischen Wirkstoffsuche speziell im HTS (High Throughput Screening).

Das Photoprotein mtClytin eignet sich als Komponente von Detektionssystemen speziell für ELISA (enzyme-linked immunosorbent assay), für Immunohistochemie, für Western-Blot, für die konfokale Mikroskopie.

Das Photoprotein mtClytin eignet sich als Marker für die Analyse von Wechselwirkungen speziell für Protein-Protein-Wechselwirkungen, für DNA-Protein-Wechselwirkungen, für DNA-RNA-Wechselwirkungen, für RNA-RNA-Wechselwirkungen, für RNA-Protein-Wechselwirkungen (DNA: deoxyribonucleic acid; RNA: ribonucleic acid; ).

Das Photoprotein mtClytin eignet sich als Marker oder Fusionsprotein für die Expression in transgenen Organismen speziell in Mäusen, in Ratten, in Hamstern und anderen Säugetieren, in Primaten, in Fischen, in Würmern, in Pflanzen.

Das Photoprotein mtClytin eignet sich als Marker oder Fusionsprotein zur Analyse der Embryonalentwicklung.

Das Photoprotein mtClytin eignet sich als Marker über einen Kopplungsvermittler speziell über Biotin, über NHS (N-hydroxysulfosuccimide), über CN-Br.

Das Photoprotein mtClytin eignet sich als Reporter gekoppelt an Nukleinsäuren speziell an DNA, an RNA.

Das Photoprotein mtClytin eignet sich als Reporter gekoppelt an Proteine oder Peptide.

Das Photoprotein mtClytin eignet sich als Reporter zur Messung von intra- oder extrazellulären Calziumkonzentrationen.

Das Photoprotein mtClytin eignet sich zur Charakterisierung von Signalkaskaden in zellulären Systemen.

Das an Nukleinsäuren oder Peptiden gekoppelte Photoprotein mtClytin eignet sich als Sonde speziell für Northern-Blots, für Southern-Blots, für Western-Blots, für ELISA, für Nukleinsäuresequenzierungen, für Proteinanalysen, Chip-Analysen.

Das Photoprotein mtClytin eignet sich zur Markierung von pharmakologischen Formulierungen speziell von infektiösen Agentien, von Antikörpers, von "small molecules".

Das Photoprotein mtClytin eignet sich für geologische Untersuchungen speziell für Meeres-, Grundwasser- und Flussströmungen.

Das Photoprotein mtClytin eignet sich zur Expression in Expressionssystemen speziell in in-vitro Translationssystemen, in bakteriellen Systemen, in Hefe Systemen, in Bakulo Systemen, in viralen . Systemen, in eukaryotischen Systemen.

Das Photoprotein mtClytin eignet sich zur Visualisierung von Geweben oder Zellen bei chirurgischen Eingriffen speziell bei invasiven, bei nicht-invasiven, bei minimal-invasiven.

Das Photoprotein mtClytin eignet sich auch zur Markierung von Tumorgeweben und anderen phänotypisch veränderten Geweben speziell bei der histologischen Untersuchung, bei operativen Eingriffen.

Beschrieben ist auch die Reinigung des Photoprotein mtClytin speziell als wildtyp Protein, als Fusionsprotein, als mutagenisiertes Protein.

Beschrieben ist auch die Reinigung des Signalpeptide von mtClytin speziell als wildtyp Protein, als Fusionsprotein, als mutagenisiertes Protein.

Beschrieben ist auch die Verwendung des Photoprotein mtClytin auf dem Gebiet der Kosmetik speziell von Badezusätzen, von Lotionen, von Seifen, von Körperfarben, von Zahncreme, von Körperpudern.

Beschrieben ist auch die Verwendung des Photoprotein mtClytin zur Färbung speziell von Nahrungsmitteln, von Badezusätzen, von Tinte, von Textilien, von Kunststoffen.

Beschrieben ist such die Verwendung des Photoprotein mtClytin zur Färbung von Papier speziell von Grußkarten, von Papierprodukten, von Tapeten, von Bastelartikeln.

Beschrieben ist auch die Verwendung des Photoprotein mtClytin zur Färbung von Flüssigkeiten speziell für Wasserpistolen, für Springbrunnen, für Getränke, für Eis.

Beschrieben ist auch die Verwendung des Photoprotein mtClytin zur Herstellung von Spielwaren speziell von Fingerfarbe, von Schminke.

Die Erfindung betrifft Nukleinsäuremoleküle, die das Polypeptid offenbart durch SEQ ID NO: 2 kodieren.

Die Erfindung betrifft das Polypeptid mit der Aminosäuresequenz, die in SEQ ID NO: 2 offenbart ist.

Beschrieben sind des weiteren auf Nukleinsäuremoleküle, ausgewählt aus der Gruppe bestehend aus
a) Nukteinsäuremolekülen, die ein Polypeptid kodieren, welches die Aminosäuresequenz offenbart durch SEQ ID NO: 2 beinhaltet;
b) Nukleinsäuremolekülen, welche die durch SEQ ID NO: 1 dargestellte Sequenz enthalten;
c) Nukleinsäuremolekülen, deren komplementärer Strang mit einem Nukleinsäuremolekül aus a) oder b) unter stringenten Bedingungen hybridisiert und welche ein Polypeptid kodieren, das die biologische Punktion eines Photoproteins aufweist;
   Eine stringente Hybridisierung von Nukleinsäuremolekülen kann zum Beispiel in einer wässrigen Lösung, die 0,2 x SSC (lx standard saline-citrate = 150 mM NaCl, 15 mM Trinatriumcitrat) enthält, bei 68°C durchgeführt werden (Sambrook et al., 1989).
d) Nukleinsäuremolekülen, welche sich auf Grund der Degenerierung des genetischen Kodes von den unter c) genannten unterscheiden;
e) Nukleinsäuremolekülen, welche eine Sequenzhomologie von mindestens 95 % zu SEQ ID NO: 1 zeigen, und deren Proteinprodukt die biologische Funktion eines Photoproteins aufweist; und
f) Nukleinsäuremolekülen, welche eine Sequenzhomologie von mindestens 65 % zu SEQ ID NO: 1 zeigen, und deren Proteinprodukt die biologische Funktion eines Photoproteins aufweist.

Beschrieben sind auch Nukleinsäuremoleküle, die eine Sequenzhomologie von mindestens 95 %, 90 %, 85 %, 80 %, 75 %, 70 %, 65 % oder 60 % zu SEQ ID NO: 1 aufweisen und für ein Polypeptid kodieren, welches die Eigenschaften eines Photoproteins besitzt.

Die Erfindung betrifft die erfindungsgemäβen Nukleinsäuremoleküle, bei denen die Sequenz einen funktionalen Promotor 5' zu der das Photoprotein kodierenden Sequenz bzw. der das Leader- oder Siganlsequenz kodierenden Sequenz enthält.

Die Erfindung betrifft auch die erfindungsgemäβen Nukleinsäuremoleküle, die Bestandteil von rekombinanten DNA oder RNA Vektoren sind.

Die Erfindung betrifft nicht-humane Organismen, die einen solchen Vektor enthalten.

Die Erfindung betrifft Photoproteine, die durch die erfindungsgemäβen Nukleotidsequenzen kodiert sind.

Die Erfindung bezieht sich auf Verfahren zur Expression der erfindungsgemäßen Photoprotein Polypeptide in Bakterien, eukaryontischen Zellen oder in in vitro Expressionssystemen.

Beschrieben sind Peptide mit mehr als 5 aufeinanderfolgenden Aminosäuren, die immunologisch durch Antikörper gegen die erfindungsgemäßen Photoproteine erkannt werden.

Die Erfindung betrifft die Verwendung der erfindungsgemäßen, für Photoproteine kodierende Nukleinsäuren als Marker- oder Reportergene, insbesondere für die pharmakologische Wirkstoffsuche und Diagnostik.

Die Erfindung betrifft die Verwendung der erfindungsgemäßen Photoproteine bzw. eine erfindungsgemäße, für ein Photoprotein kodierende Nukleinsaüre als Marker oder Reporter bzw. als Marker- oder Reportergen.

Die Erfindung betrifft die Verwendung des Photoproteins mtClytin (SEQ ID NO: 2) bzw. die Verwendung einer für das Photoprotein mtClytin kodierenden Nukleinsäure als Marker oder Reporter bzw. als Marker oder Reportergen insbesondere für die pharmakologische Wirkstoffsuche und Diagnostik.

Die Erfindung betrifft die Verwendung der in SEQ ID NO: 1 dargestellten Nukleinsäure als Marker- oder Reportergen, insbesondere für die pharmakologische Wirkstoffsuche und Diagnostik.

Beschrieben sind auch polyklonale oder monoklonale Antikörper, welche ein erfindungsgemäßes Polypeptid erkennen.

Beschrieben sind auch monoklonale oder polyklonale Antikörper, die das Photoprotein mtClytin (SEQ ID NO:2) erkennen.

### Expression der erfindungsgemäßen Photoproteine

Als Expression bezeichnet man die Produktion eines Moleküls, das nach dem Einbringen des Gens in eine geeignete Wirtszelle die Transcription und Translation des in einen Expressionsvektor klonierte Fremdgen erlaubt. Expressionsvektoren enthalten die für die Expression von Genen in Zellen von Prokaryonten oder Eukaryonten erforderlichen Kontrollsignale.

Expressionsvektoren können prinzipiell auf zwei verschiedene Weisen konstruiert werden. Bei den sogenannten Transkriptionsfusionen wird das vom einklonierten Fremdgen codierte Protein als authentisches, biologisch aktives Protein synthetisiert. Der Expressionsvektor trägt hierzu alle zur Expression benötigten 5'- und 3'- Kontrollsignale.

Bei den sogenannten Translationsfusionen wird das vom einklonierten Fremdgen codierte Protein als Hybridprotein zusammen mit einem anderen Protein exprimiert, das sich leicht nachweisen lässt. Die zur Expression benötigten 5'- und 3'- Kontrollsignale inklusive des Startcodons und eventuell ein Teil der für die N-terminalen Bereiche des zu bildenden Hybridproteins codierenden Sequenzen stammen vom Vektor. Der zusätzliche eingeführte Proteinteil stabilisiert nicht nur in vielen Fällen das vom einklonierten Fremdgen codierte Protein vor dem Abbau durch zelluläre Proteasen, sondern lässt sich auch zum Nachweis und zur Isolierung des gebildeten Hybridproteins einsetzen. Die Expression kann sowohl transient, als auch stabil erfolgen. Als Wirtsorganismen eignen sich sowohl Bakterien, Hefen, Viren als auch eukaryotische Systeme.

### Reinigung der erfindungsgemäßen Photoproteine

Die Isolierung von Proteinen (auch nach Überexpression) wird häufig als Proteinreinigung bezeichnet. Zur Proteinreinigung steht eine Vielzahl an etablierten Methoden und Verfahren zur Verfügung.

Die Fest-Flüssig-Trennung ist eine Grundoperation bei Proteinisolierungen. Sowohl bei der Abtrennung der Zellen vom Kulturmedium als auch bei der Klärung des Rohextraktes nach Zellaufschluss und Entfernung der Zelltrümmer, bei der Abtrennung von Niederschlägen nach Fällungen usw. ist der Verfahrensschritt erforderlich. Er erfolgt durch Zentrifugation und Filtration.

Durch Gewinnung intrazellulärer Proteine muss die Zellwand zerstört bzw. durchlässig gemacht werden. Je nach Maßstab und Organismus werden dazu Hochdruckhomogenisatoren oder Rührwerkskugel- bzw. Glasperlenmühlen eingesetzt. Im Labormaßstab kommen u.a. mechanische Zellintegrationen und Ultraschallbehandlung zum Einsatz.

Sowohl für extrazelluläre als auch intrazelluläre Proteine (nach Zellaufschluss) sind verschiedene Fällungsverfahren mit Salzen (insbesondere Ammoniumsulfat) oder organischen Lösungsmitteln (Alkohole, Aceton) eine schnelle und effiziente Methode zur Konzentration von Proteinen. Bei der Reinigung intrazellulärer Proteine ist die Entfernung der löslichen Nukleinsäuren erstrebenswert (Fällung z.B. mit Streptomycin- oder Protaminsulfat). Bei der Gewinnung extrazellulärer Proteine werden häufig Träger (z.B. Stärke, Kieselgur) vor Zugabe der Fällungsmittel zugesetzt, um besser handhabbare Niederschläge zu erhalten.

Für die Feinreinigung stehen zahlreiche chromatographische und Verteilungsverfahren zur Verfügung (Absorptions- und Ionenaustauschchromatographie, Gelfiltration, Affinitätschromatographie, Elektrophoresen). Eine Säulenchromatographie wird auch im technischen Maßstab angewandt. Für den Labormaßstab ist vor allem die Affinitätschromatographie von Bedeutung, die Reinigungsfaktoren bis zu mehreren 100 pro Schritt ermöglicht.

Extrazelluläre Proteine fallen in relativ verdünnten Lösungen an. Sie müssen ebenso wie extrazelluläre Proteine vor ihrer weiteren Verwendung konzentriert werden. Neben den schon erwähnten Verfahren hat sich - auch im industriellen Maßstab - die Ultrafiltration bewährt.

Anorganische Salze als Begleitstoffe von Proteinen sind für spezifische Anwendungen häufig unerwünscht. Sie können u.a. durch Gelfiltration, Dialyse und Diafiltration entfernt werden.

Zahlreiche Proteine kommen als Trockenpräparate zum Einsatz. Als Trocknungsverfahren sind die Vakuum-, Gefrier- und Sprühtrocknung von Bedeutung.

### Nukleotid- und Aminosäuresequenzen

Das Photoprotein mtClytin wird durch die folgende Nukleotidsequenz kodiert (SEQ ID NO: 1):

Daraus ergibt sich eine Aminosäuresequenz von (SEQ ID NO: 2):

Das putative Signalpeptide des Photoprotein mtClytin besitzt folgende Sequenz (SEQ ID NO: 3):
MQRFTNRLLSMSALRA
und weist folgende Nukleinsäuresequenz auf:
5'- atgcaaaggtttacaaatcgtcttctttccatgtcggctttacgtgca - 3' (SEQ ID NO 4)

Diese Sequenzen finden sich im Sequenzlisting wieder.

### Kurze Beschreibung der Figuren

- Figur 1:: Die Figur 1 zeigt die Plasmidkarte des Vektors pTrip1EX2-mtClytin.
- Figur 2:: Die Figur 2 zeigt die Plasmidkarte des Vektors pcDNA3-mtClytin.
- Figur 3:: Die Figur 3 zeigt das Ergebnis der bakteriellen Expression von mtClytin, sowie die Biolumineszenzaktivität von mtClytin nach bakterieller Expression. (Y = RLU : relative light units; X = Verdünnung; schwarze Balken = mtClytin; graue Balken = Kontrolllysat).
- Figur 4:: Die Figur 4 zeigt das Ergebnis der eukaryotische Expression von mtClytin, sowie die Biolumineszenzaktivität von mtClytin nach Expression in CHO Zellen. (Y = RLU : relative light units; X = ATP (logarithmische Darstellung in mol/l)).
- Figur 5:: Die Fig. 5 zeigt die kinetische Analyse der Biolumineszenz von mtClytin. (Y = RLU : relative light units; X = Zeit [Sekunden]).
- Figur 6:: Die Fig. 6 zeigt die kinetische Analyse der Biolumineszenz von Obelin. (Y = RLU : relative light units; X = Zeit [Sekunden]).
- Figur 7 :: Die Figur 7 zeigt das Aligment von Clytin und mtCyltin auf Aminosäureebene.
- Figur 8 :: Die Figur 8 zeigt das Aligment von Clytin und mtCyltin auf Nukleinsäureebene.
- Figur 9 :: Die Figur 9 zeigt das Aligment von Clytin, mtCyltin und Clytin-2 auf Aminosäureebene.

### Beispiele

### Beispiel 1

Als Vektor zur Herstellung des im folgenden dargestellten Konstruktes wurde das Plasmid pTriplEx2 der Firma Clontech verwendet. Das Derivat des Vektors wurde als pTriplEx2-mtClytin bezeichnet. Der Vektor pTriplEx2-mtClytin wurde zur Expression von mtClytin in bakteriellen Systemen verwendet.

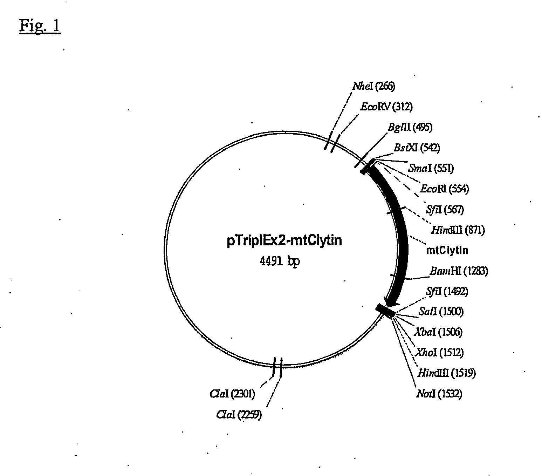

Die Figur 1 zeigt die Plasmidkarte des Vektors pTriplEX2-mtClytin

### Beispiel 2

Als Vektor zur Herstellung des im folgenden dargestellten Konstruktes wurde das Plasmid pcDNA3.1(+) der Firma Clontech verwendet. Das Derivat des Vektors wurde als pcDNA3-mtClytin bezeichnet. Der Vektor pcDNA3-mtClytin wurde zur Expression von mtClytin in eukaryotischen Systemen verwendet.

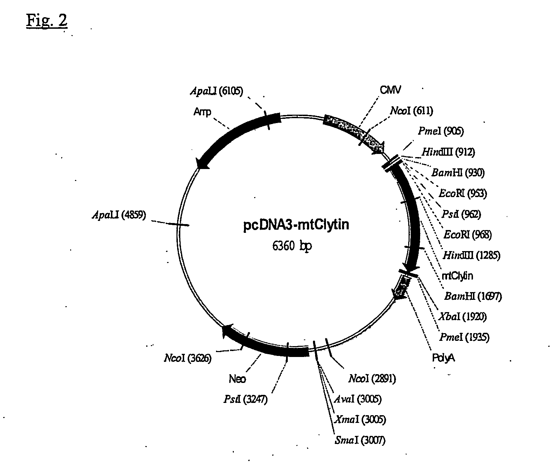

Die Figur 2 zeigt die Plasmidkarte des Vektors pcDNA3-mtClytin.

### Beispiel 3

### Bakterielle Expression

Die bakterielle Expression erfolgte im E. coli Stamm BL21(DE3) durch Transformation der Bakterien mit den Expressionsplasmiden pTriplEX2-mtClytin und pTriplEX2. Die transformierten Bakterien wurden in LB-Medium bei 37°C für 3 Stunden inkubiert und die Expression für 4 Stunden durch Zugabe von IPTG bis zu einer Endkonzentration von 1 mM induziert. Die induzierten Bakterien wurden durch Zentrifugation geerntet, in 50 mM Tris/HCl (pH 9,0) + 5 mM EDTA resuspendiert und durch Ultraschall aufgeschlossen. Das Lysat wurde anschließend für 15 Minuten bei 13000 Umdrehungen pro Minute (16000 rcf) zentrifugiert und der Überstand abgenommen. Der Überstand (Verdünnungen 1:5; 1:10; 1:20 und 1:50 mit Tris/HCl pH 9,0)) wurde 3 Stunden mit Coelenterazin (10E-07 M Coelenterazine in Tris/HCl pH 9,0) im dunkeln inkubiert. Direkt nach der Zugabe von 5 mM Calziumchlorid wurde die Biolumineszenz im Luminometer gemessen. Die Integrationszeit der Messung betrug 40 Sekunden.

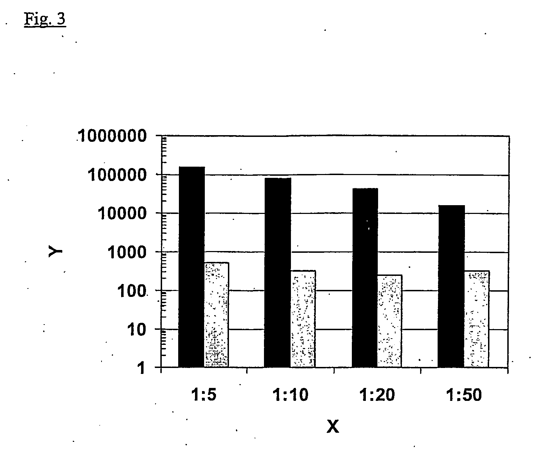

Die Figur 3 zeigt die Ergebnisse der Biolumineszenzmessung von mtClytin in Bakterien.

### Beispiel 4

### Eukaryotische Expression

Die konstitutive eukaryotische Expression erfolgte in CHO-Zellen durch Transfektion der Zellen mit den Expressionsplasmiden pcDNA3-mtClytin und pcDNA3.1(+) in transienten Experimenten. Hierzu wurden 10000 Zellen pro Loch in DMEM-F12 Medium auf 96 Loch Mikrotiterplatten plattiert und über Nacht ber 37°C inkubiert. Die Transfektion erfolgte mit Hilfe des Fugene 6 Kits (Roche) nach Herstellerangaben. Die transfizierten Zellen wurden über Nacht bei 37°C in DMEM-F12 Medium inkubiert. Anschließend wurde das Medium entfernt und durch 50 µl Coelenterazin (10E-07 M Coelenterazine in PBS) ersetzt. Die Zellen wurden für 3 Stunden bei 37°C inkubiert und anschließend ATP (Adenosintriphosphat) bis zu einer Finalkonzentration von 1 µM zugegeben. Die Messung wurde direkt nach der Zugabe im Luminometer gestartet. Die Integrationszeit betrug 1 Sekunde, bei einer Gesamtmessdauer von 60 Sekunden.

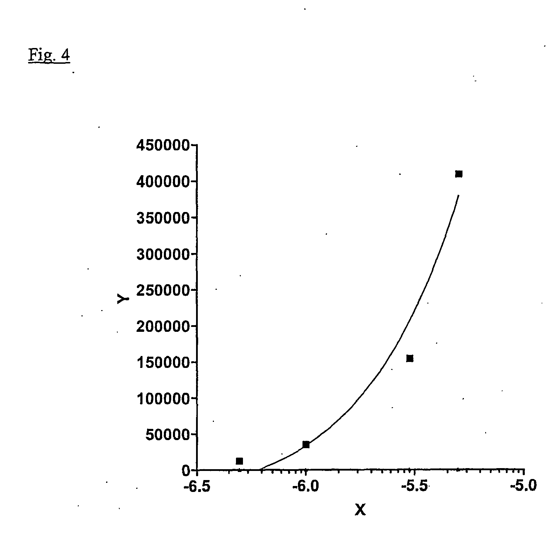

Die Figur 4 zeigt die Ergebnisse der Biolumineszenzmessung von mtClytin in CHO Zellen.

### Beispiel 5

### BLAST

Ergebnis einer BLAST-Analyse von mtClytin auf der Aminosäureebene.
>emb|CAD87655.1| unnamed protein product [Clytia gregaria], Length = 198, Score = 368 bits (945), Expect = e-101, Identities = 171/195 (87%), Positives = 182/195 (92%)
>sp|Q08121|CLYT_CLYGR Clytin precursor (Phialidin), pir||S28860 clytin - hydromedusa (Clytia gregarium), emb|CAA49754.1| clytin [Clytia gregaria], gb|AAA28293.1| apoclytin, Length = 198, Score = 368 bits (945), Expect = e-101, Identities = 171/195 (87%), Positives = 182/195 (92%)
>emb|CAD87658.1| unnamed protein product [synthetic construct], Length = 198, Score = 367 bits (943), Expect = e-101, Identities = 170/195 (87%), Positives = 182/195 (93%)
>sp|Q27709|OBL_OBELO Obelin precursor (OBL), pdb|1EL4|A Chain A, Structure Of The Calcium-Regulated Photoprotein Obelin, Determined By Sulfur Sas, gb|AAA67708.1| unnamed protein product, Length = 195, Score = 327 bits (837), Expect = 1e-88, Identities = 150/193 (77%), Positives = 170/193 (87%)
>emb|CAD87674.1| unnamed protein product [synthetic construct], Length = 195, Score = 326 bits (835), Expect = 2e-88, Identities = 149/193 (77%), Positives = 170/193 (87%)
>emb|CAD87672.1| unnamed protein product [synthetic construct], Length = 195, Score = 325 bits (834), Expect = 3e-88, Identities = 149/193 (77%), positives = 170/193 (87%)
>emb|CAD87673.1| unnamed protein product [synthetic construct], Length = 195, Score = 325 bits (833), Expect = 4e-88, Identities = 149/193 (77%), Positives = 170/193 (87%)
>pdb|1JF0|A Chain A, The Crystal Structure Of Obelin From Obelia Geniculata At 1.82 A Resolution, gb|AAL86372.1|AF394688_1 apoobelin [Obelia geniculata], Length = 195, Score = 325 bits (833), Expect = 4e-88, Identities = 149/193 (77%), Positives = 168/193 (86%)

### Beispiel 6

### BLAST

Ergebnis einer BLAST-Analyse von mtClytin auf Nukleinsäureebene :
>emb|AX702125.1| Sequence 23 from Patent WO03006497, Length = 597, Score = 669 bits (348), Expect = 0.0, Identities = 504/582 (86%)
>emb|AX702119.1| Sequence 17 from Patent WO03006497, Length = 597, Score = 669 bits (348), Expect = 0.0, Identities = 504/582 (86%)
>emb|X70221.1|CGCLYTIN C.gregaria mRNA for clytin, Length = 747, Score = 669 bits (348), Expect = 0.0, Identities = 504/582 (86%)
>gb|L13247.1|CY1APOCLYT Clytia gregarium apoclytin mRNA, complete cds, Length = 747, Score = 669 bits (348), Expect = 0.0, Identities = 504/582 (86%)
>emb|AX702187.1| Sequence 85 from Patent WO03006497, Length = 597, Score = 664 bits (345), Expect = 0.0, Identities = 503/582 (86%)
>emb|AX702185.1| Sequence 83 from Patent WO03006497, Length = 597, Score = 664 bits (345), Expect = 0.0, Identities = 503/582 (86%)
>emb|AX702183.1| Sequence 81 from Patent WO03006497, Length = 597, Score = 664 bits (345), Expect = 0.0, Identities = 503/582 (86%)
>emb|AX702181.1| Sequence 79 from Patent WO03006497, Length = 597, Score = 664 bits (345), Expect = 0.0, Identities = 503/582 (86%)
>emb|AX702179.1| Sequence 77 from Patent WO03006497, Length = 597, Score = 664 bits (345), Expect = 0.0, Identities = 503/582 (86%)
>emb|AX702131.1| Sequence 29 from Patent WO03006497, Length = 597, Score = 664 bits (345), Expect = 0.0, Identities = 503/582 (86%)
>emb|AX702129.1| Sequence 27 from Patent WO03006497, Length = 597, Score = 664 bits (345), Expect = 0.0, Identities = 503/582 (86%)

### Beispiel 7

Die Figur 7 zeigt das Alignment von mtClytin mit Clytin (Clytia gregaria) auf Nukleinsäureebene.

### Beispiel 8

Die Figur 8 zeigt das Alignment von mtClytin mit Clytin (Clytia gregaria) auf Aminosäureebene.

### Beispiel 9

### Kinetische Analyse von mtClytin

Zur kinetischen Analyse der Biolumineszenz von mtClytin, wurden CHO Zellen mit pcDNA3-mtClytin bzw. pcDNA-Obelin oder pcDNA3 (ohne integrierte cDNA) transient transfiziert. Die Transfektion und Messung erfolgte wie unter Beispiel 4-beschrieben. Die Messdaten wurden für einen Zeitraum von 60 Sekunden mit einer Integrationszeit von 1 Sekunde erhoben.

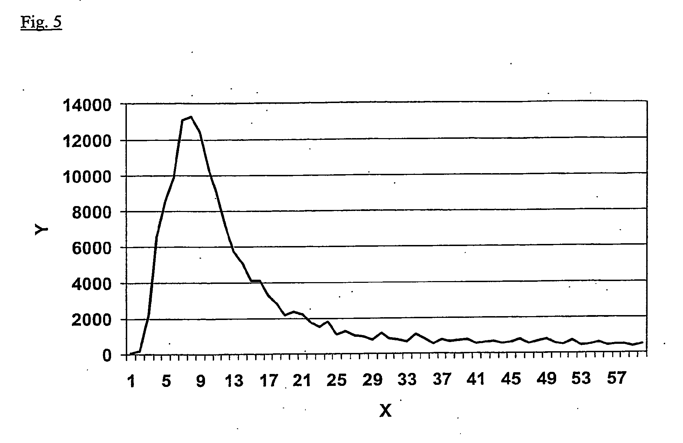

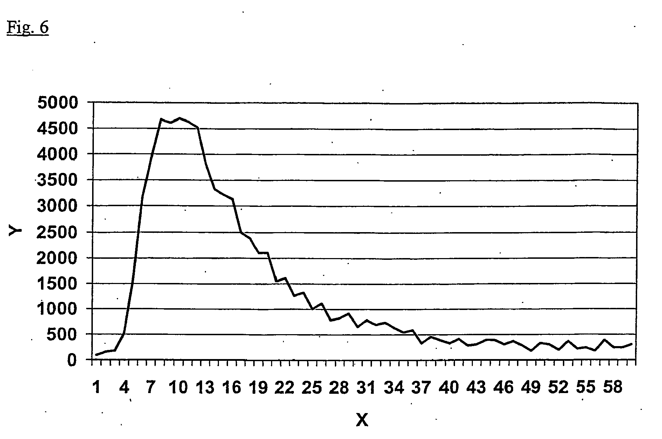

Die Figuren 5 und 6 zeigen die Ergebnisse der kinetischen Analyse von mtClytin und Obelin.

### Beispiel 10

### MITOPROT-Analyse

Zur Analyse des Signalpeptides von mtClytin wurde das Computerprogramm MTTOPROT verwendet (Claros et al., 1996). Folgende Photoproteine wurden analysiert: Obelin (Q27709), Aequorin (P07164), Clytin (Q08121) und mtClytin (SEQ ID NO. 2).

### Ergebnisse der Analysen:

### Obelin:

| | | | | |
|---|---|---|---|---|
| Sequence name: OBELIN | | | | |
| Input sequence length : 195 aa | | | | |

| VALUES OF COMPUTED PARAMETERS | | | | |
|---|---|---|---|---|
| Net charge of query sequence : | | | | -11 |
| Analysed region : | | | | 11 |
| Number of basic residues in targeting sequence : | | | | 3 |
| Number of acidic residues in targeting sequence : | | | | 0 |
| Cleavagesite : | not predictable | | | |
| Cleaved sequence : | - | | | |

| HYDROPHOBIC SCALE USED | | | | |
|---|---|---|---|---|
| | GES | KD | GVH1 | ECS |
| H17 : | -0.624 | 0.259 | -0.308 | 0.295 |
| MesoH : | -1.573 | -0.241 | -0.642 | 0.060 |
| MuHd_075 : | 14.019 | 3.641 | 4.408 | 1.523 |
| MuHd_095 : | 7.994 | 7.898 | 3.285 | 1.838 |
| MuHd_100 : | 13.734 | 9.836 | 5.597 | 2.742 |
| MuHd_105 : | 21.195 | 11.755 | 7.339 | 4.117 |
| Hmax_075 : | -9.450 | -2.800 | -4.008 | 1.132 |
| Hmax_095 : | -0.963 | 1.837 | -1.971 | 1.103 |
| Hmax_100 : | 0.400 | 1.300 | -1.942 | 2.240 |
| Hmax_105 : | 10.617 | 6.067 | 0.733 | 3.127 |

| PROBABILITY | | | | |
|---|---|---|---|---|
| of export to mitochondria: 0.1479 | | | | |
| Aequorin : | | | | |
| Sequence name: AEQUORIN | | | | |
| Input sequence length : 196 aa | | | | |

| VALUES OF COMPUTED PARAMETERS | | | | |
|---|---|---|---|---|
| Net charge of query sequence : | | | | -13 |
| Analysed region : | | | | 3 |
| Number of basic residues in targeting sequence : | | | | 0 |
| Number of acidic residues in targeting sequence : | | | | 0 |
| Cleavage site : | not predictable | | | |
| Cleaved sequence : | - | | | |

| HYDROPHOBIC SCALE USED | | | | |
|---|---|---|---|---|
| | GES | KD | GVH1 | ECS |
| H17 : | 0.006 | 0.794 | -0.263 | 0.368 |
| MesoH : | -1.673 | -0.382 | -0.703 | 0.048 |
| MuHd_075 : | 24.326 | 4.153 | 5.947 | 2.450 |
| MuHd_095 : | 12.638 | 7.213 | 4.218 | 1.796 |
| MuHd_100 : | 13.748 | 8.827 | 4.477 | 2.427 |
| MuHd_105 : | 16.581 | 11.426 | 5.056 | 3.453 |
| Hmax_075 : | 0.438 | 0.233 | -2.490 | 1.692 |
| Hmax_095 : | 0.525 | -1.400 | -2.394 | 0.674 |
| Hmax_100 : | -0.100 | -1.200 | -2.292 | 1.550 |
| Hmax_105 : | 0.500 | -0.000 | -2.164 | 1.540 |

| PROBABILITY | | | | |
|---|---|---|---|---|
| of export to mitochondria: 0.0148 | | | | |
| Clytin : | | | | |
| Sequence name: CLYTIN | | | | |
| Input sequence length : 198 aa | | | | |

| VALUES OF COMPUTED PARAMETERS | | | | |
|---|---|---|---|---|
| Net charge of query sequence : | | | | -11 |
| Analysed region : | | | | 11 |
| Number of basic residues in targeting sequence : | | | | 3 |
| Number of acidic residues in targeting sequence : | | | | 0 |
| Cleavagesite : | not predictable | | | |
| Cleaved sequence : | - | | | |

| HYDROPHOBIC SCALE USED | | | | |
|---|---|---|---|---|
| | GES | KD | GVH1 | ECS |
| H17 : | -0.624 | 0.259 | -0.308 | 0.295 |
| MesoH : | -1.573 | -0.241 | -0.642 | 0.060 |
| MuHd_075 : | 14.019 | 3.641 | 4.408 | 1.523 |
| MuHd_095 : | 7.994 | 7.898 | 3.285 | 1.838 |
| MuHd_100 : | 13.734 | 9.836 | 5.597 | 2.742 |
| MuHd_105 : | 21.195 | 11.755 | 7.339 | 4.117 |
| Hmax_075 : | -9.450 | -2.800 | -4.008 | 1.132 |
| Hmax_095 : | -0.963 | 1.837 | -1.971 | 1.103 |
| Hmax_100 : | 0.400 | 1.300 | -1.942 | 2.240 |
| Hmax_105 : | 10.617 | 6.067 | 0.733 | 3.127 |

| PROBABILITY | | | | |
|---|---|---|---|---|
| of export to mitochondria: 0.1479 | | | | |
| Aequorin : | | | | |
| Sequence name: AEQUORIN | | | | |
| Input sequence length : 196 aa | | | | |

| VALUES OF COMPUTED PARAMETERS | | | | |
|---|---|---|---|---|
| Net charge of query sequence : | | | | -13 |
| Analysed region : | | | | 3 |
| Number of basic residues in targeting sequence : | | | | 0 |
| Number of acidic residues in targeting sequence : | | | | 0 |
| Cleavage site : | not predictable | | | |
| Cleaved sequence : | - | | | |

| HYDROPHOBIC SCALE USED | | | | |
|---|---|---|---|---|
| | GES | KD | GVH1 | ECS |
| H17 : | 0.006 | 0.794 | -0.263 | 0.368 |
| MesoH : | -1.673 | -0.382 | -0.703 | 0.048 |
| MuHd_075 : | 24.326 | 4.153 | 5.947 | 2.450 |
| MuHd_095 : | 12.638 | 7.213 | 4.218 | 1.796 |
| MuHd_100 : | 13.748 | 8.827 | 4.477 | 2.427 |
| MuHd_105 : | 16.581 | 11.426 | 5.056 | 3.453 |
| Hmax_075 : | 0.438 | 0.233 | -2.490 | 1.692 |
| Hmax_095 : | 0.525 | -1.400 | -2.394 | 0.674 |
| Hmax_100 : | -0.100 | -1.200 | -2.292 | 1.550 |
| Hmax_105 : | 0.500 | -0.000 | -2.164 | 1.540 |

| PROBABILITY | | | | |
|---|---|---|---|---|
| of export to mitochondria: 0.0148 | | | | |
| Clytin : | | | | |
| Sequence name: CLYTIN | | | | |
| Input sequence length : 198 aa | | | | |

| VALUES OF COMPUTED PARAMETERS | | | | |
|---|---|---|---|---|
| Net charge of query sequence : | | | | -9 |
| Analysed region : | | | | 32 |
| Number of basic residues in targeting sequence : | | | | 6 |
| Number of acidic residues in targeting sequence : | | | | 2 |
| Cleavage site : | not predictable | | | |
| Cleaved sequence : | - | | | |

| HYDROPHOBIC SCALE USED | | | | |
|---|---|---|---|---|
| | GES | KD | GVH1 | ECS |
| H17 : | -0.429 | 0.341 | -0.313 | 0.313 |
| MesoH : | -1.778 | -0.307 | -0.718 | 0.053 |
| MuHd_075 : | 32.928 | 17.509 | 7.351 | 5.708 |
| MuHd_095 : | 30.874 | 20.344 | 9.074 | 5.834 |
| MuHd_100 : | 36.596 | 22.666 | 10.051 | 6.762 |
| MuHd_105 : | 39.174 | 19.336 | 10.379 | 7.609 |
| Hmax_075 : | 4.900 | 7.087 | -1.223 | 3.684 |
| Hmax_095 : | 13.600 | 10.100 | 1.251 | 4.390 |
| Hmax_100 : | 14.000 | 12.600 | 1.601 | 5.060 |
| Hmax_105 : | 6.650 | 13.067 | -0.468 | 3.920 |

| PROBABILITY | | | | |
|---|---|---|---|---|
| of export to mitochondria: 0.2047 | | | | |
| Clytin-2 : | | | | |
| Sequence name: CLYTIN-2 | | | | |
| Input sequence length : 198 aa | | | | |

| VALUES OF COMPUTED PARAMETERS | | | | |
|---|---|---|---|---|
| Net charge of query sequence : | | | | -7 |
| Analysed region : | | | | 16 |
| Number of basic residues in targeting sequence : | | | | 3 |
| Number of acidic residues in targeting sequence : | | | | 1 |
| Cleavage site : | not predictable | | | |
| Cleaved sequence : | - | | | |

| HYDROPHOBIC SCALE USED | | | | |
|---|---|---|---|---|
| | GES | KD | GVH1 | ECS |
| H17 : | -0.288 | 0.341 | -0.213 | 0.313 |
| MesoH : | -1.519 | -0.206 | -0.681 | 0.081 |
| MuHd_075 : | 32.594 | 15.092 | 8.192 | 4.075 |
| MuHd_095 : | 36.090 | 19.707 | 8.836 | 6.716 |
| MuHd_100 : | 38.617 | 20.269 | 9.682 | 6.851 |
| MuHd_105 : | 30.267 | 16.082 | 8.229 | 5.470 |
| Hmax_075 : | 6.533 | 6.417 | -0.793 | 2.508 |
| Hmax_095 : | 13.600 | 10.100 | 1.251 | 4.390 |
| Hmax_100 : | 13.600 | 10.100 | 1.251 | 4.390 |
| Hmax_105 : | 13.417 | 10.150 | 1.612 | 3.862 |

| PROBABILITY | | | | |
|---|---|---|---|---|
| of export to mitochondria: 0.3974 | | | | |
| mtClytin : | | | | |
| Sequence name: mtClytin | | | | |
| Input sequence length : 228 aa | | | | |

| VALUES OF COMPUTED PARAMETERS | | | | |
|---|---|---|---|---|
| Net charge of query sequence : | | | | -8 |
| Analysed region : | | | | 34 |
| Number of basic residues in targeting sequence : | | | | 6 |
| Number of acidic residues in targeting sequence : | | | | 0 |
| Cleavage site : | | 17 | | |
| Cleaved sequence : | | MQRFTNRLLSMSALRA | | |

| HYDROPHOBIC SCALE USED | | | | |
|---|---|---|---|---|
| | GES | KD | GVH1 | ECS |
| H17 : | -0.135 | 0.453 | -0.343 | 0.309 |
| MesoH : | -1.623 | -0.215 | -0.701 | 0.073 |
| MuHd_075 : | 33.394 | 19.322 | 8.634 | 7.593 |
| MuHd_095 : | 34.726 | 19.634 | 8.110 | 8.861 |
| MuHd_100 : | 32.825 | 16.596 | 7.376 | 7.520 |
| MuHd_105 : | 28.005 | 19.893 | 7.410 | 7.865 |
| Hmax_075 : | 16.683 | 17.733 | 2.851 | 5.763 |
| Hmax_095 : | 13.125 | 13.388 | 2.299 | 4.314 |
| Hmax_100 : | 8.300 | 11.500 | 1.845 | 3.830 |
| Hmax_105 : | 1.700 | 9.500 | -1.171 | 2.390 |

| PROBABILITY | | | | |
|---|---|---|---|---|
| of export to mitochondria: 0.9974 | | | | |

Die Wahrscheinlichkeit einer Translokation des analysierten Peptides in Mitochondrien steigt mit der Annäherung des berechneten Faktors an 1.

Die. Analyse der Proteinsequenzen von Obelin, Aequorin, Clytin, Clytin-2 und mtClytin hat ergeben, dass nur mtClytin die Merkmale eines Proteins aufweist, dass in Mitochondrien transportiert werden kann.

### Beispiel 11

Die Figur 9 zeigt das Alignment von mtClytin, Clytin (Clytia gregaria) und Clytin-type2 auf Aminosäureebene.

### Literatur / Patente

US 6,495,355
US 5,541,309
US 5,093,240
US-0908909
US 6,152,358
JP-0176125
GB-0024357
WO03006497
WO200168824
Alam J, Cook JL. Reporter genes: application to the study of mammalian gene transcription. Anal Biochem. 1990 Aug 1;188(2):245-54
Altschul, Stephen F., Thomas L. Madden, Alejandro A. Schäffer, Jinghui Zhang, Zheng Zhang, Webb Miller, and David J. Lipman (1997); Gapped BLAST and PSI-BLAST: a new generation of protein database search programs; Nucleic Acids Res. 25:3389-3402
Chiesa A, Rapizzi E, Tosello V, Pinton P, de Virgilio M, Fogarty KE, Rizzuto R. Recombinant aequorin and green fluorescent protein as valuable tools in the study of cell signalling. Biochem J. 2001 Apr 1;355(Pt 1):1-12.
Claros, M.G., Vincens, P. (1996); Computational method to predict mitochondrially imported proteins and their targeting seqeunces. Eur. J. Biochem 241, 779-786.
Cullen Bryan R., Malim Michael H., Secreted placental alkaline phosphatase as a eukaryotic reporter gene. Methods in Enzymology. 216:362ff
Fagan TF, Ohmiya Y, Blinks JR, Inouye S, Tsuji FI. Cloning, expression and sequence analysis of cDNA for the Ca(2+)-binding photoprotein, mitrocomin. FEBSLett. 1993 Nov 1;333(3):30.1-5
Hastings, J.W. and Morin, J.G. (1969) Comparative biochemistry of calcium-activated photoproteins from the ctenophore, Mnemiopsis and the coelenterates Aequorea, Obelia, and Pelagia. Biol. Bull. 137, 402.
Haddock SH, Rivers TJ, Robison BH. Can coelenterates make coelenterazine? Dietary requirement for luciferin in cnidarian bioluminescence. Proc Natl Acad Sci U S A 2001 Sep 25;98(20):11148-51
Inouye S, Tsuji FI. (1994) Aequorea green fluorescent protein. Expression of the gene and fluorescence characteristics of the recombinant protein. FEBS Lett 1994 Mar 21;341(2-3):277-80
Inouye S, Tsuji FI. Cloning and sequence analysis of cDNA for the Ca(2+)-activated photoprotein, clytin. FEBS Lett. 1993 Jan 11;315(3):343-6.
Illarionov BA, Bondar VS, Illarionova VA, Vysotski ES. Sequence of the cDNA encoding the Ca(2+)-activated photoprotein obelin from the hydroid polyp Obelia longissima. Gene. 1995 Feb 14;153(2):273-4.
Jones K, Hibbert F, Keenan M. Glowing jellyfish, luminescence and a molecule called coelenterazine. Trends Biotechnol 1999 Dec;17(12):477-81
Johnson, F.H., Shimomura, O., Saiga, Y., Gershman, L.C., Reynolds, G.T., and Waters, J.R. (1962) Quantum efficiency of Cypridina luminescence, with a note on that of Aequorea. J. Cell. Comp. Physiol. 60, 85-103.
Morin, J.G. and Hastings, J.W. (1971) Biochemistry of the bioluminescence of colonial hydroids and other coelenterates. J. Cell. Physiol. 77, 305-311.
Phillips GN, Structure and dynamics of green fluorescent protein. Curr Opin Struct Biol. 1997 Dec;7(6):821-7
Sambrook, J., Fritsch, E. Maniatis, T. 1989, Molecular cloning. A laboratory manual Vol 1-3, Cold Spring Harbor, New York : Cold Spring Harbor Laboratory Press
Shimomura O, Johnson FH. Properties of the bioluminescent protein aequorin. Biocheinisry. 1969 Oct;8(10):3991-7
Shimomura O., Bioluminescence in the sea: photoprotein systems. Symp Soc Exp Biol. 1985;39:351-72
Shimomura O. Isolation and properties of various molecular forms of aequorin. Biochem J. 1986 Mar 1;234(2):271-7.
Snowdowne KW, Borle AB. Measurement of cytosolic free calcium in mammalian cells with aequorin. Am J Physiol. 1984 Nov;247(5 Pt 1):C396-408.
Ward, W.W. (1998) Biochemical and physical properties of green fluorescent protein. In: Green Fluoreseent Protein: Properties, Applications, and Protocols (Chalfie, M. and Kain, S., eds) pp. 45-70. Wiley-Liss, Inc.
Yang Te-Tuan, Sinai Parisa, Kitts Paul A. Kain Seven R., Quantification of gene expresssion with a secreted alkaline phosphatase reporter system. Biotechnique. 1997 23(6) 1110ff

## Patentansprüche

1. Nukleinsäuremolekül, ausgewählt aus der Gruppe bestehend aus
a) Nukleinsäuremolekülen, die ein Polypeptid kodieren, welches die Aminosäuresequenz offenbart durch SEQ ID NO: 2 beinhaltet; und
b) Nukleinsäuremolekülen, welche die in SEQ ID NO: 1 dargestellte Sequenz beinhalten.

2. Nukleinsäure nach Anspruch 1, welche einen funktionalen Promotor 5' zur kodierenden Sequenz enthält.

3. Rekombinante DNA oder RNA Vektoren, welche Nukleinsäuren nach Anspruch 2 enthalten.

4. Nicht humane Organismen, die einen Vektor gemäß Anspruch 3 enthalten.

5. Peptid oder Polypeptid, das durch eine Nukleinsäuresequenz nach Anspruch 1 kodiert ist.

6. Verfahren zur Expression der Polypeptide gemäß Anspruch 5 in Bakterien, viralen Systemen, Hefen oder eukaryontischen Zellen oder in *in vitro* Expressionssystemen.

7. Verfahren zur Aufreinigung/Isolierung eines Photoprotein Polypeptides gemäß Anspruch 6.

8. Verwendung einer Nukleinsäure gemäß den Ansprüchen 1 bis 3 als Marker- oder Reportergen.

9. Verwendung eines Polypeptids gemäß Anspruch 5 als Marker oder Reporter.

10. Verwendung eines Polypeptides gemäß Anspruch 5 als Reporterprotein in der pharmakologischen irkstoffsuche.

11. Verwendung einer Nukleinsäure gemäß Ansprüchen 1 oder 2 als Reportergen in der pharmakologischen Wirkstoffsuche.

## Claims

1. A nucleic acid molecule selected from the group consisting of
a) nucleic acid molecules, which encode a polypeptide which contains the amino acid sequence disclosed by SEQ ID NO: 2; and
b) nucleic acid molecules which contain the sequence depicted by SEQ ID NO: 1 :

2. The nucleic acid as claimed in claim 1, which contains a functional promoter 5' to its coding sequence.

3. A recombinant DNA or RNA vector, which contains nucleic acids according to claim 2.

4. Non-human organisms, which harbor a vector as claimed in claim 3.

5. A peptide or polypeptide, which is encoded by a nucleic acid sequence as claimed in claim 1.

6. A method for expressing the polypeptides as claimed in claim 5 in bacteria, viral systems, yeasts, or eukaryotic cells or in *in-vitro* expression systems.

7. A method for purifying/isolating a photoprotein polypeptide as claimed in claim 6.

8. The use of a nucleic acid as claimed in claim 1 to 3 as a marker gene or reporter gene.

9. The use of a polypeptide as claimed in claim 5 as a label or reporter.

10. The use of a polypeptide as claimed in claim 5 as reporter protein in searching for pharmacological active compounds.

11. The use of a nucleic acid as claimed in claim 1 or 2 as reporter gene in searching for pharmacological active compounds.

## Revendications

1. Molécule d'acide nucléique, sélectionnée parmi le groupe consistant en
a) des molécules d'acide nucléique qui encodent un polypeptide qui contient la séquence d'acide aminé exposée par SEQ ID NO : 2 ; et
b) des molécules d'acide nucléique qui contiennent la séquence représentée par SEQ ID NO : 1.

2. Acide nucléique selon la revendication 1 qui contient un promoteur fonctionnel 5' de la séquence encodant.

3. Vecteurs d'ADN ou d'ARN recombinants qui contiennent des acides nucléiques selon la revendication 2.

4. Organismes non humains qui contiennent un vecteur selon la revendication 3.

5. Peptide ou polypeptide qui est encodé par une séquence d'acide nucléique selon la revendication 1,

6. Procédé d'expression des polypeptides selon la revendication 5 dans des bactéries, des systèmes viraux, des levures ou dans des cellules eucaryotes ou dans des systèmes d'expression in vitro.

7. Procédé de purification/d'isolation d'un photoprotéine polypeptide selon la revendication 6.

8. Utilisation d'un acide nucléique selon la revendication 1 à 3 comme gène marqueur ou gène rapporteur,

9. Utilisation d'un polypeptide selon la revendication 5 comme marqueur ou rapporteur.

10. Utilisation d'un polypeptide selon la revendication 5 comme protéine rapporteur dans la recherche des agents actifs pharmacologiques.

11. Utilisation d'un acide nucléique selon l'une des revendications 1 ou 2 comme gène rapporteur dans la recherche des agents actifs pharmacologiques.
